# EUROPEAN PATENT SPECIFICATION

(11) **EP 3 898 910 B1**
(45) Date of publication and mention of the grant of the patent: **03.09.2025**
(21) Application number: 18826662.1
(22) Date of filing: 20.12.2018
(51) Int. Cl.: C11B 9/00, C07C 69/716, C07D 307/16

(54) **ALICYCLIC MUSK FRAGRANCE COMPOUNDS**
ALICYCLISCHE MOSCHUSRIECHSTOFFVERBINDUNGEN
COMPOSÉS ALICYCLIQUES DE PARFUM MUSQUÉ

(43) Date of publication of application: 27.10.2021
(73) Proprietor: Symrise AG, 37603 Holzminden Niedersachsen (DE)
(72) Inventor: HÖLSCHER, Bernd, 37620 Halle (DE); CHANDRASEKARAN, Vijayanand, 37603 Holzminden (DE); KIERMASCH, Eva, 37603 Holzminden (DE); WAGNER, Tobias, 37627 Hellental (DE)
(74) Representative: Eisenführ Speiser
(86) International application number: PCT/EP2018/086190
(87) International publication number: WO 2020/125993

(56) References cited:
- EP-A1- 0 472 966
- EP-A1- 1 398 366
- WO-A1-00/14051
- WO-A1-2008/049257
- WO-A1-2012/055875
- FR-A1- 2 008 167

## Description

The present invention primarily relates to the use of a compound selected from the group consisting of 1-(3,3-dimethylcyclohexyl)ethyl 4-oxopentanoate, 2-(1-(3,3-dimethylcyclohexyl)ethoxy)-2-methylpropyl tetra-hydrofuran-2-carboxylate, 1-(3,3-dimethylcyclohexyl)ethyl 4-oxoheptanoate, 1-(3,3-dimethylcyclohexyl)ethyl 4-oxooctanoate and 2-(1-(3,3-dimethylcyclohexyl)ethoxy)-2-oxoethyl 4-oxopentanoate, Moreover, the present invention relates to perfume compositions and perfumed products comprising the before mentioned perfuming ingredients. Still more particularly, the invention relates to a method for producing said perfumed products and a method of imparting and/or increasing musk odor characteristics to perfumed products.

Although many perfumes are already available, in the perfume industry there is still a general demand for new perfumes especially for perfumes with musk fragrance notes, which are able (in perfume compositions) to produce, in addition to a musk fragrance note, other interesting odor notes and/or odor impressions and, with their novel or original fragrance properties, to expand the possibilities of the perfumer. In particular there is interest in perfumes with musk fragrance notes that are able to form a harmonious combination with perfumes with a flowery fragrance. Preferably there should be an overlapping of the different olfactory aspects and notes, so as to produce a complex overall odor impression.

The area of perfume chemistry can be considered to be well-researched in the prior art. In the area of musk perfumes, we may mention in particular Helvetolide/Serenolide, Romandolide and Appelide and derivatives thereof.

Musk perfumes from the stated groups are described in documents WO 2012/055875 A1, DE 102 14 675 A1,

WO 2004/050602 A1, EP 0 472 966 A1, WO 00/14051 A1, EP 1 262 474 A1, EP 2 632 554 A1 and EP 1 398 366 A1.

The odor descriptions of the known perfumes, from our own investigations and from the patent literature, can be summarized as follows:
1. Helvetolide (Firmenich): strong musk note of ambrette type. Molecular weight: 284
2. Serenolide (Givaudan): strong musk note. Molecular weight: 296
3. Romandolide (Firmenich): woody, musk-like, of nitro type. Molecular weight: 270
4. Appelide (IFF): musk-like, of nitro type, fruity Molecular weight: 256

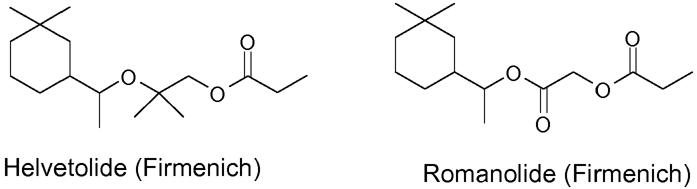

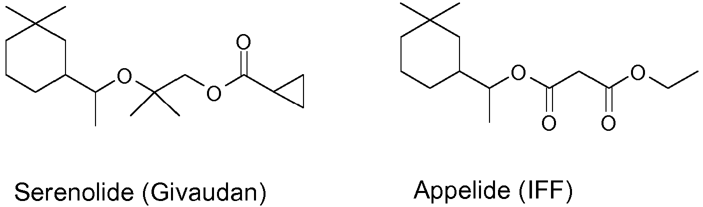

Work on these classes of compounds relating to derivatization of the side chain was not pursued further in the prior art. This can probably be attributed to the earlier teaching that perfumes with a molecular weight above 294 (exception: Serenolide with 296) are not of interest as perfumes (see G. Ohloff Scent and Fragrances, the Fascination of Odors and their Chemical Perspectives, Springer Verlag Berlin 1994 p. 9). A person skilled in the art would therefore assume that other derivatives of these basic structures, with higher molecular weight, lead to impairment of the olfactory properties.

The search for suitable perfumes, which led to the present invention, was made difficult by the following circumstances:
- The mechanisms of odor perception are not adequately known.
- The relations between special odor perception on the one hand and the chemical structure of the associated perfume on the other hand have not been investigated sufficiently.
- Just slight changes in the structure of a known perfume often cause large changes in the sensory properties and impair compatibility for the human organism.
- The sensory effects of interactions with other perfumes cannot be predicted.

The success of a search for suitable perfumes is therefore strongly dependent on the intuition of the person conducting the search.

Against this background, the problem to be solved by the present invention was to provide perfume compounds which, as well as a musk note, also possess fruity notes (aspects) and/or intensify such notes.

A further problem to be solved by the present invention was to provide perfume compounds with a high stability under specified conditions of use.

In addition to these main problems, the perfume compounds that are to be provided preferably have, as well as their primary (olfactory) properties, an additional or more preferably more than one additional positive secondary properties, for example good adherence, high substantivity, a booster effect also for other than flowery perfumes, a strong blooming and/or the property of imparting and/or intensifying other desirable (subsidiary) odor notes or odor impressions.

According to the invention, the primary problem is solved with a compound selected from the group consisting of 1-(3,3-dimethylcyclohexyl)ethyl 4-oxopentanoate, 2-(1-(3,3-dimethylcyclohexyl)ethoxy)-2-methylpropyl tetra-hydrofuran-2-carboxylate, 1-(3,3-dimethylcyclohexyl)ethyl 4-oxoheptanoate, 1-(3,3-dimethylcyclohexyl)ethyl 4-oxooctanoate and 2-(1-(3,3-dimethylcyclohexyl)ethoxy)-2-oxoethyl 4-oxopentanoate.

The compounds according to the invention are shown in the following table 1:

**Table 1**

| **No.:** | **Structure** | **Name** | **Odor** |
|---|---|---|---|
| **1** | 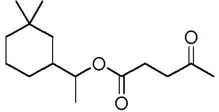 | 1-(3,3-dimethylcyclohexyl)ethyl 4-oxopentanoate | Musky, more floral, rubber, fruity, apple, honey, leather. |
| **2** | 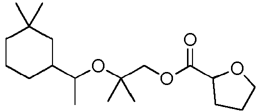 | 2-(1-(3,3-dimethylcyclohexyl)etho xy)-2-methylpropyl tetrahydrofuran-2-carboxylate | Musk, Clean, more fruity |
| **3** | 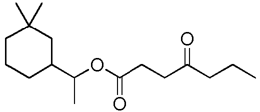 | 1-(3,3-dimethylcyclohexyl)ethyl 4-oxoheptanoate | Musk, clean, soft, milky, coconut, milky |
| **4** | 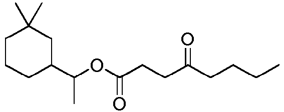 | 1-(3,3-dimethylcyclohexyl)ethyl 4-oxooctanoate | Musk, fruity, leathery. |
| **5** | 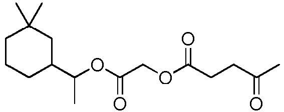 | 2-(1-(3,3-dimethylcyclohexyl)etho xy)-2-oxoethyl 4-oxopentanoate | Musk, fruity, powdery |

A compound according to the invention is especially preferred that is selected from the group consisting of 2-(1-(3,3-dimethylcyclohexyl)ethoxy)-2-methylpropyl tetrahydrofuran-2-carboxylate, 1-(3,3-dimethylcyclohexyl)ethyl 4-oxoheptanoate, 1-(3,3-dimethylcyclohexyl)ethyl 4-oxooctanoate, and 2-(1-(3,3-dimethylcyclohexyl)ethoxy)-2-oxoethyl 4-oxopentanoate.

As well as the odor notes and olfactory properties described above, musk and fruity, the especially preferred compounds possess in particular the further odor notes / olfactory properties mentioned in Table 1.

The compounds according to the invention can be in optically active form and even in isomerically pure form. They can, however, also be used as any mixture of the stereoisomers, in particular also as racemates.

The compounds according to the invention can be used in combination with one or more of the following compounds (1) to (6): 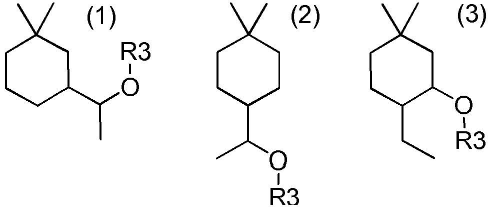 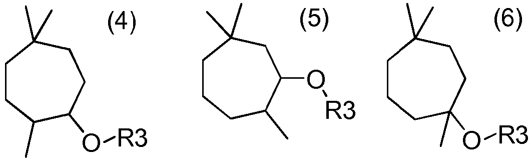 wherein R3 has the following general formula (IV) 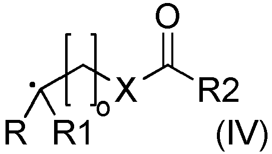 and wherein o is 0 or 1, X is O or methylene, R and R1 are each methyl or form together with the carbon atom attached to a carbonyl group, and R2 is methyl, ethyl, propyl, butyl, butan-2-one-4-yl, tetrahydrofuran-2-yl, or tetrahydrofuran-3-yl.

Mixtures of compounds according to the invention and one or more of the compounds (1) to (6) can be used to enhance, modify or impart a musk and fruity odor.

The various olfactory properties could not be predicted for the compounds according to the invention from the prior art. This applies in particular to the combination of musk notes, fruity aspects (notes).

In addition to the primary (olfactory) properties, the compounds according to the invention possess additional positive secondary properties. In particular, relative to similar compounds from the prior art, we may mention improved adherence and high substantivity.

Many of the compounds according to the invention have a molecular weight above 300, so that for the reasons stated above, the industry assumed that molecules of this size do not include any suitable fragrances.

Compounds according to the invention with a molecular weight (MW) ≥ 300 are preferred in many cases. These compounds possess surprisingly strong olfactory properties, with excellent adherence and/or high substantivity.

Especially 2-(1-(3,3-dimethylcyclohexyl)ethoxy)-2-methylpropyl tetrahydrofuran-2-carboxylate (compound 2) with an molecular weight of 326 g/mol shows a very strong musk odor and is the highest molecular weight alicyclic musk known until now.

It is also surprising that the compounds according to the invention and in particular the aforementioned preferred compounds according to the invention show an enhanced stability in comparison with romandolide as a benchmark under normal use condition of perfuming compounds.

It has also been surprisingly found that the compounds according to the invention and in particular the aforementioned preferred compounds according to the invention show long lasting tenacity of above 7.8.

Furthermore, it is surprising that the compounds according to the invention possess positive properties such as substantivity, adhesion and biodegradability, but at the same time do not have any unpleasant odor notes, even as a result of hydrolysis.

These properties, too, could not be predicted based on the modifications, which were undertaken contrary to the prior art.

Further aspects of the invention can be seen from the following description, the examples, and the appended patent claims.

The present invention also relates to a use of a compound selected from the group consisting of 1-(3,3-dimethylcyclohexyl)ethyl 4-oxopentanoate, 2-(1-(3,3-dimethylcyclohexyl)ethoxy)-2-methylpropyl tetra-hydrofuran-2-carboxylate, 1-(3,3-dimethylcyclohexyl)ethyl 4-oxoheptanoate, 1-(3,3-dimethylcyclohexyl)ethyl 4-oxooctanoate and 2-(1-(3,3-dimethylcyclohexyl)ethoxy)-2-oxoethyl 4-oxopentanoate, as a perfuming ingredient.

A perfuming ingredient is, in the context of the present text, any substance that is suitable for being used for bringing about an olfactory impression, i.e. for imparting an olfactory impression, or for altering (modifying or intensifying) the olfactory perception of another substance.

"Impart" a note or an odor characteristic is to be understood as follows, in the sense of this application:
A perfume mixture does not possess the note to be imparted or an odor characteristic to be imparted. Following addition of a sufficient amount of the compound according to the invention or of a mixture of the compounds according to the invention, the corresponding notes (the corresponding odor characteristic) are sensorially perceptible. In this case an imparting is present.

"Intensify" is to be understood as follows:
The comparative mixture V already possesses a corresponding note / a corresponding odor characteristic. On adding the compound according to the invention or a mixture of compounds according to the invention in a sufficient amount, the corresponding odor characteristic / the corresponding odor note is sensorially perceptibly intensified.

In case of doubt, the presence of imparting and/or intensifying is to be established by a panel of experts (e.g. 10 experts) with sensory training. Intensification or imparting of an impression is present when a corresponding effect is established sensorially, reproducibly by at least 75% of the panellists.

The use according to the invention is preferred, for enhancing, modifying or imparting a musk odor and/or for enhancing, modifying or imparting a fruity odor.

In accordance with the foregoing, another aspect of the invention relates in particular to a perfume composition comprising at least one compound according to the present invention.

Perfume compositions according to the invention are usually liquid at 25°C and 1013hPa and normally are homogeneous solutions.

Perfume compositions often comprise synthetic or natural (preferably) taste and odor neutral carrier oils, which contain the scent or fragrance substance (as artificial or natural substances) in highly concentrated form (as well as perfumistic solvents and/or auxiliary materials, if applicable). The same applies accordingly to the perfume compositions according to the invention described herein.

Preferably, the perfume composition according to the invention comprises one or more additional fragrance ingredients.

Examples for fragrance ingredients that generally preferably can be used as component of a perfume composition according to the invention can be found for example in S. Arctander, Perfume and Flavor Chemicals, Vol. I and II, Montclair, N. J., 1969, author's edition or H. Surburg, J. Panten, Common Fragrance and Flavor Materials, 5th Ed., Wiley-VCH, Weinheim 2006.

Preferred ethereal oils, concretes, absolutes, resins, resinoids, balsams and/or tinctures, that can be a fragrance ingredient of a perfume composition according to the invention are preferably to be selected from the group consisting of:
Ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tee oil; bitter almond oil; savory oil; bucco leaf oil; cabreuva oil; cade oil; calmus oil; camphor oil; cananga oil; cardamom oil; cascarilla oil; cassia oil; cassie absolute; castoreum absolute; cedar leaf oil; cedar wood oil; cistus oil; citronella oil; citrus oil; copaiva balsam; copaiva balsam oil; coriander oil; costus root oil; cumin oil; cypress oil; davana oil; dill weed oil; dill seed oil; eau de brouts absolute; oakmoss absolute; elemi oil; estragon oil; eucalyptus citriodora oil; eucalyptus oil; fennel oil; spruce needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calamus oil; chamomile oil blue; chamomile oil roman; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil squeezed; linaloe oil; litsea cubeba oil; bay leaf oil; macis oil; Marjoram oil; mandarin oil; massoia bark oil; mimosa absolute; musk seed oil; musk tincture; muscatel sage oil; nutmeg oil; myrrh absolute; myrrh oil; myrtle oil; carnation leaf oil; carnation blossom oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; allspice oil; pine oil; poley oil; rose absolute; rosewood oil; rose oil; rosemary oil; sage oil Dalmatian; sage oil Spanish; sandalwood oil; celery seed oil; spike lavender oil; star aniseed oil; styrax oil; tagetes oil; fir needle oil; tea tree oil; turpentine oil; thyme oil; tolu balsam; tonka absolute; tuberose absolute; vanilla extract; violet leaf absolute; verbena oil; vetiver oil; juniper berry oil; wine yeast oil; vermouth oil; wintergreen oil; ylang oil; ysop oil; civet absolute; cinnamon leaf oil; cinnamon bark oil.

Preferred single fragrance substances that can be preferably used as fragrance ingredients of a perfume composition according to the invention are selected from the group of
hydrocarbons, thereby preferred 3-carene; a-pinene; b-pinene; a-terpinenes; g-terpinenes; p-cymene; bisabolene; camphene; caryophyllene; cedrene; farnesene; limonene; longifolene; myrcene; ocimene; valencene; (E,Z)-1,3,5-undecatriene; styrene; diphenylmethane;
aliphatic alcohols, thereby preferred hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
aliphatic aldehydes and their acetals, thereby preferred hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyl oxyacetaldehyde; 1-(1-methoxy-propoxy)-(E/Z)-3-hexene;
aliphatic ketones and their oximes, thereby preferred 2-heptanone; 2-octanone; 3-octanone; 2-nonanone; 5-methyl-3-heptanone; 5-methyl-3-heptanone oxime; 2,4,4,7-tetramethyl-6-octene-3-one; 6-methyl-5-heptene-2-one;
aliphatic sulphur-containing compounds, thereby preferred 3-methylthio-hexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
aliphatic nitriles, thereby preferred 2-nonenoic acid nitrile; 2-undecenoic acid nitrile; 2-tridecenoic acid nitril; 3,12-tridecadienoic acid nitrile; 3,7-dimethyl-2,6-octadienoic acid nitrile; 3,7-dimethyl-6-octenoic acid nitril;
esters of aliphatic carboxylic acids, thereby preferred (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl-isobutyrate; hexyl crotonate; ethyl isovalerate; ethyl-2-methylpentanoate; ethyl hexanoate; allyl hexanoate; ethyl heptanoate; allyl heptanoate; ethyl octanoate; ethyl-(E,Z)-2,4-decadienoate; methyl-2-octinate; methyl-2-noninate; allyl-2-isoamyl oxyacetate; methyl-3,7-dimethyl-2,6-octadienoate;4-methyl-2-pentyl-crotonate;
acyclic terpene alcohols, thereby preferred citronellol; geraniol; Nerol; linalool; lavadulol; nerolidol; farnesol; tetrahydrolinalool; tetrahydrogeraniol; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol 2,6-dimethyl-2,5,7-octatrien-1-ol; as well as their formates, acetates, propionates, isobutyrates, butyrates, isovalerianates, pentanoates, hexanoates, crotonates, tiglinates and 3-methyl-2-butenoates;
acyclic terpene aldehydes and -ketones, thereby preferred geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethyl acetals of geranial, neral, 7-hydroxy-3,7-dimethyloctanal;
cyclic terpene alcohols, thereby preferred isopulegol; alpha-terpineol; terpinenol-4; menthan-8-ol; menthan-1-ol; menthan-7-ol; borneol; isoborneol; linalool oxide; nopol; cedrol; ambrinol; vetiverol; guaiol; as well as their formates, acetates, propionates, isobutyrates, butyrates, isovalerianates, pentanoates, hexanoates, crotonates, tiglinates and 3-methyl-2-butenoates; menthyl formate; menthyl propionate; menthyl butyrate; menthyl isobutyrate; menthyl isovalerianate; menthyl hexanoate; menthyl crotonate; menthyl tiglinate;
cyclic terpene aldehydes and -ketones, thereby preferred menthone; isomenthone; 8-mercaptomenthan-3-one; carvone; camphor; fenchone; alpha-ionone; beta-ionone; beta-n-methyl ionone; beta-isomethyl ionone; alpha-irone; alpha-damascone; beta-damascone; beta-damascenone; delta-damascon; gamma-damascon; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedarwood oil (methyl cedryl ketone);
cyclic alcohols, thereby preferred 4-tert.-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
cycloaliphatic alcohols, thereby preferred alpha,3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
cyclic and cycloaliphatic ethers, thereby preferred cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo[10.1.0]trideca-4,8-diene; rose oxide; 2-(2,4-dimethyl-3-cyclohexen-1-yl)-5-methyl-5-(1-methylpropyl)-1,3-dioxane;
cyclic and macrocyclic ketones, thereby preferred 4-tert.-butyl cyclohexanone; 2,2,5-trimethyl-5-pentyl cyclopentanone; 2-heptyl cyclopentanone; 2-pentyl cyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert.-pentylcyclohexanone; 5-cyclohexadecen-1-one; 6,7-dihydro-1, 1,2,3,3-pentamethyl-4(5H)-indanone; 8-cyclohexadecen-1-one; 9-cycloheptadecen-1-one; cyclopentadecanone; cyclohexadecanone;
cycloaliphatic aldehydes, thereby preferred 2,4-dimethyl-3-cyclohexene carbaldehyde; 2-methyl-4-(2,2,6-trimethyl-cyclohexen-1-yl)-2-butenal; 4-(4-hydroxy-4-methylpentyl)-3-cyclohexene carbaldehyde; 4-(4-methyl-3-penten-1-yl)-3-cyclohexene carbaldehyde;
cycloaliphatic ketones, thereby preferred 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphtalenylmethylketone; methyl-2,6,10-trimethyl-2,5,9-cyclododecatrienylketone; tert.-butyl-(2,4-dimethyl-3-cyclohexen-1-yl)ketone;
esters of cyclic alcohols, thereby preferred 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5, or 6-indenyl acetate; 4,7-methano-3a,4,5,6,7,7a-hexahydro-5, or 6-indenyl propionate; 4,7-methano-3a,4,5,6,7,7a-hexahydro-5, or 6-indenyl isobutyrate; 4,7-methanooctahydro-5, or 6-indenyl acetate;
esters of cycloaliphatic alcohols, preferably 1-cyclohexylethyl crotonate;
esters of cycloaliphatic carboxylic acids, thereby preferred allyl-3-cyclohexyl propionate; allyl cyclohexyloxy acetate; cis- and trans-methyldihydrojasmonate; cis- and trans-methyljasmonate; methyl-2-hexyl-3-oxocyclopentane carboxylate; ethyl-2-ethyl-6,6-dimethyl-2-cyclohexene carboxylate; ethyl-2,3,6,6-tetramethyl-2-cyclohexene carboxylate; ethyl-2-methyl-1,3-dioxolane-2-acetate;
araliphatic alcohols, thereby preferred benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentan-1-ol; 3-methyl-5-phenylpentanol; 3-phenyl-2-propen-1-ol; 4-methoxybenzyl alcohol; 1-(4-isopropylphenyl)ethanol;
esters of araliphatic alcohols and aliphatic carboxylic acids, thereby preferred benzyl acetate; benzyl propionate; benzyl isobutyrate; benzyl isovalerianate; 2-phenylethyl acetate; 2-phenylethyl propionate; 2-phenylethyl isobutyrate; 2-phenylethyl isovalerianate; 1-phenylethyl acetate; alpha-trichloromethyl benzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
araliphatic ethers, thereby preferred 2-phenylethyl methyl ether; 2-phenylethyl isoamyl ether; 2-phenylethyl-1-ethoxyethyl ether; phenyl acetaldehyde dimethyl acetal; phenyl acetaldehyde diethyl acetal; hydratropic aldehyde dimethyl acetal; phenyl acetaldehyde glyceryl acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
aromatic and araliphatic aldehydes, thereby preferred benzaldehyde; phenyl acetaldehyde; 3-Phenyl propanal; hydratropic aldehyde; 4-methyl benzaldehyde; 4-methylphenyl acetaldehyde; 3-(4-ethylphenyl)-2,2-dimethyl propanal; 2-methyl-3-(4-isopropylphenyl)propanal; 2-methyl-3-(4-tert.-butylphenyl)propanal; 2-methyl-3-(4-isobutylphenyl)propanal; 3-(4-tert.-butylphenyl)propanal; cinnamic aldehyde; alpha-butyl cinnamic aldehyde; alpha-amyl cinnamic aldehyde; alpha-hexyl cinnamic aldehyde; 3-methyl-5-phenylpentanal; 4-methoxy benzaldehyde; 4-hydroxy-3-ethoxy benzaldehyde; 3,4-methylendioxy benzaldehyde; 3,4-dimethoxy benzaldehyde; 2-methyl-3-(4-methoxyphenyl)propanal; 2-methyl-3-(4-methylendioxyphenyl)propanal;
aromatic and araliphatic ketones, thereby preferred acetophenone; 4-methylacetophenone; 4-methoxyacetophenone; 4-tert.-butyl-2,6-dimethylacetophenone; 4-phenyl-2-butanone; 4-(4-hydroxyphenyl)-2-butanone; 1-(2-naphthalenyl)ethanone; 2-benzofuranylethanone; (3-methyl-2-benzofuranyl)ethanone; benzophenone; 1,1,2,3,3,6-hexamethyl-5-indanylmethylketone; 6-tert.-butyl-1,1-dimethyl-4-indanylmethylketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
aromatic and araliphatic carboxylic acids and their esters, thereby preferred benzoic acid; Phenylacetic acid; methyl benzoate; ethyl benzoate; hexyl benzoate; methyl phenylacetate; ethyl phenylacetate; geranyl phenylacetate; phenylethyl-phenyl acetate; methyl cinnamate; ethyl cinnamate; benzyl cinnamate; phenylethyl cinnamate; cinnamyl cinnamate; allylphenoxy acetate; methyl salicylate; isoamyl salicylate; hexyl salicylate; cyclohexyl salicylate; cis-3-hexenyl salicylate; benzyl salicylate; phenylethyl salicylate; methyl-2,4-dihydroxy-3,6-dimethyl benzoate; ethyl-3-phenylglycidate; ethyl-3-methyl-3-phenylglycidate;
nitrogen-containing aromatic compounds, thereby preferred 2,4,6-trinitro-1,3-dimethyl-5-tert.-butylbenzene; 3,5-dinitro-2,6-dimethyl-4-tert.-butyl acetophenone; cinnamic acid nitrile; 3-methyl-5-phenyl-2-pentenoic acid nitrile; 3-methyl-5-phenylpentanoic acid nitrile; methyl anthranilate; methy-N-methyl anthranilate; Schiff bases of methyl anthranilate with 7-hydroxy-3,7-dimethyloctanal, 2-methyl-3-(4-tert.-butylphenyl)propanal or 2,4-dimethyl-3-cyclohexene carbaldehyde; 6-isopropyl chinolin; 6-isobutyl chinolin; 6-sec.-butyl chinolin; 2-(3-phenylpropyl)pyridine; indole; skatole; 2-methoxy-3-isopropyl pyrazine; 2-isobutyl-3-methoxy pyrazine;
phenols, phenyl ethers and phenyl esters, thereby preferred estragol; anethol; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenyl acetate;
heterocyclic compounds, thereby preferred 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
lactones, thereby preferred 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene-1,13-tridecandioate; coumarin; 2,3-dihydrocoumarin; octahydrocoumarin.

The special properties of the compounds according to the invention can - as already mentioned - be exploited particularly well in order to produce interesting and desirable fragrance notes/properties in perfume compositions according to the invention.

The compounds according to the invention are usually employed in sensorially effective amounts. In perfume compositions, the compounds according to the invention are mixed with further perfumes. These further perfumes can in principle be any known perfumes.

A perfume composition according to the invention is preferred in which the mass ratio of the total of the perfumes according to the invention to the total of the further perfumes contained in the perfume mixture is 1:1000 to 1:05, preferably 1:25 to 1:100.

At this ratio, the advantages of the compounds according to the invention can be exploited particularly well.

A perfume composition according to the invention in which the proportion of the total of the perfumes according to the invention is 0.00001 to 99.9 wt.%, preferably 0.001 to 70 wt.% and especially preferably 0.01 to 50 wt.%, relative to the total weight of the perfume composition, is further preferred according to the invention.

Accordingly, another aspect of the present invention relates to a perfumed product. A perfumed product according to the invention comprises at least one compound according to the present invention, preferably in a sensorially effective amount.

Preferred perfumed products according to the invention are detergents and cleaning products, hygiene or care products, especially products from the area of body and hair care, cosmetics and household products.

Preferred products are for example perfume extraits, eau de parfums, eau de toilettes, aftershaves, eau de colognes, pre-shave products, splash colognes and perfumed refreshing tissues as well as perfumed or to be perfumed acidic, alkaline and neutral detergents, such as e.g. floor cleaners, window glass cleaners, dishwashing detergents, bathroom and sanitary cleaners, scouring agent, solid or liquid toilet cleaners, toilet sticks, toilet stones (liquid or solid), powdery or foamy carpet cleaners, liquid detergents, powdery detergents, laundry pretreatment agents such as bleaches, soaking agents and stain removers, fabric softeners, laundry soaps, laundry tablets, disinfectants, surface disinfectants as well as air improvers in liquid or gel-like form or applied to a solid carrier, particularly for deodorization of exhaust air from air conditioning and industrial processes, as well as air improvers in the form of aerosol or pump sprays, waxes and polishes such as furniture polishes, floor waxes, shoe creams, strengthening, impregnating or deodorizing textile treatment agents, diapers, sanitary towels, panty liners, plasters, as well as personal care agents such as e.g. solid and liquid soaps, shower gels, shampoos, shaving soap, shaving foams, bathing oils, damp cleaning cloths, cosmetic emulsion of the oil-in-water, water-in-oil and water-in-oil-in-water type such as e.g. skin creams and lotions, face creams and lotions, sun protection creams and lotions, after sun creams and lotions, hand creams and lotions, foot creams and lotions, depilatory creams and lotions, aftershave creams and lotions, tanning creams and lotions, hair care products such as e.g. hair sprays, hair gels, strengthening hair lotions, hair conditioners, permanent or semi-permanent hair dyes, hair forming agents such as cold waves and hair smoothing agents, hair tonics, hair creams and lotions, deodorants and antiperspirants such as e.g. armpit sprays, roll-ons, deo sticks, deo creams, products for decorative cosmetic such as e.g. eyeshadow, makeups, lipsticks, mascara as well as candles, lamp oils, incense sticks, animal litter, cat litter, insecticides, repellents, liquid and gaseous fuels, heating oils and heating gases.

Particularly preferred perfumed products according to the invention are selected from the group consisting of perfume extraits, eau de perfumes, eau de toilettes, aftershaves, eau de colognes, pre-shave products, splash colognes, perfumed refreshing tissues, acidic, alkaline and neutral detergents, textile fresheners, ironing aids, liquid detergents, powdery detergents, laundry pretreatment agents, fabric softeners, laundry soaps, laundry tablets, disinfectants, surface disinfectants, air improvers, aerosol sprays, waxes and polishes, personal care agents, hand creams and lotions, foot creams and lotions, depilatory creams and lotions, aftershave creams and lotions, tanning creams and lotions, hair care products, deodorants and antiperspirants, products for decorative cosmetic, candles, lamp oils, incense sticks, insecticides, repellents and fuels.

For purposes of clarification it has to be mentioned that (perfumed) products according to the invention within the scope of the present text are to be understood as products that have been caused or produced on purpose, but not as naturally occurring substance mixtures, for example such as the ones that can be obtained from plant-based starting materials by means of extraction.

The compounds according to the present invention are preferably a constituent of a perfume composition according to the invention. Therefore, in a preferred embodiment, a perfumed product comprises a perfume composition according to the invention, and a carrier or a substrate, wherein the carrier or the substrate is in direct contact with said perfume composition. The substrate is for example a solid substrate or the carrier is for example a solid carrier. The carrier or substrate guarantees a fine distribution of the perfume composition inside the product as well as a controlled release during application. Such carriers may be porous inorganic materials such as silica gels, zeolites, gypsums, clay, clay granules, aerated concrete etc. or organic materials such as woods and cellulose-based substances.

The compounds according to the present invention to be used according to the invention or perfume compositions according to the present invention may also be present in microencapsulated or spray-dried form, as inclusion complexes or as extrusion products and can be added to a product in this form.

If applicable, the properties of compounds according to the present invention to be used according to the invention or of perfume compositions according to the present invention can be further optimized by means of so-called "coating" with suitable materials with regard to a more targeted release, wherein preferably wax-like plastic materials such as e.g. polyvinyl alcohol are used.

A microencapsulation of the compounds according to the present invention to be used according to the invention or perfume compositions according to the present invention can take place, for example, by means of the so-called coacervation process with the aid of capsule materials, e.g. of polyurethane-like substances or soft gelatin. Spray-dried compounds according to the present invention can be produced, for example, by means of spray-drying of a compound according to the invention, i.e. of an emulsion or dispersion containing a compound according to the present invention or a perfume compositions according to the present invention, wherein modified starches, proteins, dextrins and/or plant-based gums can be used as carrier substance. Inclusion complexes can be produced, for example, by means of addition of dispersions, which are or comprise a compound according to the present invention or a perfume compositions according to the present invention, and cyclodextrins or urea derivatives into a suitable solvent, e.g. water. Extrusion products can take place by means of fusion of a compound according to the present invention or a perfume compositions according to the present invention with a suitable wax-like substance and extrusion with subsequent solidification, in a suitable solvent, e.g. isopropanol, if applicable.

The compounds according to the present invention or perfume compositions according to the present invention can be used in many preparations or products, wherein they are preferably combined with one or several of the following excipients or active ingredients:
Preserving agents, abrasives, anti-acne agents, agents against skin aging, antibacterial agents, anticellulite agents, antidandruff agents, anti-inflammatory agents, irritation preventing agents, irritation inhibiting agents, antimicrobial agents, antioxidants, astringents, sweat inhibiting agents, antiseptic agents, antistatic agents, binders, buffers, carrier materials, chelate builders, cell stimulants, cleaning agents, caring agents, depilatory agents, surface active agents, deodorizing agents, antiperspirants, plasticizers, emulsifiers, enzymes, ethereal oils, fibres, fixators, foam builders, foam stabilizers, substances to prevent foaming, foam boosters, fungicides, gelatinizing agents, gelforming agents, hair care products, hair forming products, smoothing agents, moisturizing agents, dampening substances, moist-keeping substances, bleaching agents, (textile-)strengthening agents, stain removing agents, optical brightening agents, impregnating agents, dirt-repellent agents, friction-lowering agents, lubricants, moisturizing creams, ointments, opacifiers, plasticizing agents, covering agents, polish, glazing agents, polymers, powders, proteins, regreasing substances, abrasive agents, silicones, skin soothing agents, skin cleaning agents, skin caring agents, skin healing agents, skin lightening agents, skin protecting agents, skin softening agents, cooling agents, skin cooling agents, warming agents, skin warming agents, stabilizers, UV-absorbing agents, UV-filters, detergents, fabric softeners, suspending agents, skin tanning agents, thickening agents, vitamins, oils, waxes, fats, phospholipids, saturated fatty acids, mono- or polyunsaturated fatty acids, α-hydroxy acids, polyhydroxy fatty acids, liquefying agents, dyes, color-protecting agents, pigments, anticorrosives, aromas, flavorings, aromatic substances, polyols, surfactants, electrolytes, organic solvents or silicon derivatives.

According to one embodiment of the present invention a preferred product according to the invention, particularly a deodorant or the like, additionally contains (depending on the desired mode of action) one or several of the following active substances:
(1) antimicrobially active substances that inhibit the development of microorganisms that are responsible for the smell of perspiration; for example Triclosanâ (5-chloro-2-(2,4-dichlorophenoxy)phenol), triclocarban, chlorhexidine, chlorhexidine hydrochloride, chlorhexidine diacetate, chlorhexidine digluconate, 2-phenoxyethanol, farnesol, glycerin esters and -ethers such as glyceryl monolaurate, glyceryl monocaprinate, hexoxyglycerin, octoxyglycerin (= ethylhexylglycerin, 3-(2-ethylhexyloxy-1,2-propanediol) or Sensiva^{®} SC 50 (by Schülke & Mayr), aliphatic 1,2-diols such as e.g. 1,2-decanediol (EP 1 269 983), araliphatic alcohols such as for example described in EP 799 174, preferably 4-methyl-4-phenyl-2-pentanol (Vetikol; WO 03/024907) or 2-methyl-4-phenyl-2-butanol (1,1-dimethyl-3-phenylpropanol, alpha,alpha-dimethylphenethylcarbinol), I-menthyl methyl ether as described in WO 02/41861, 2-benzylheptan-1-ol (Jasmol; 2-n-pentyl-3-phenylpropan-1-ol), 2,2-dimethyl-3-phenylpropanol (muguet alcohol; cf. US 4,091,090), antimicrobially active secondary alcohols, such as for example described in WO 2005/004601, particularly 3-methyl-6-phenyl-2-hexanol, 4-(2,4-dimethylphenyl)-2-butanol, 6-(4-isopropylphenyl)-3-methyl-2-hexanol, 4-(2,4,5-trimethylphenyl)-2-butanol, 3,3-dimethyl-4-phenyl-2-butanol, 3-methyl-4-(2-methylphenyl)-2-butanol, 6-(3,4-dimethylphenyl)-2-hexanol, aliphatic carboxylic acids such as 2-hexyloctanoic acid, 2-hexyldecanoic acid, 2-butyloctanoic acid or 2-butyldecanoic acid;
(2) enzyme inhibiting substances that inhibit the effect of enzymes that participate in the formation of smell of perspiration; for example, citric acid esters and metal-chelating substances such as EDTA (ethylenediaminetetraacetic acid), EGTA [ethylenebis(oxyethylenenitrilo)-tetraacetic acid] and DTPA (diethylenetriaminepentaacetic acid, pentetic acid);
(3) odor absorbing substances that absorb substances that are responsible for the smell of perspiration; for example, zinc rizinoleate, cyclodextrins;
(4) antiperspirants that inhibit sweat secretion and thus eliminate the breeding grounds of bacteria that are responsible for body odor. Astringent metal salts are generally preferably used as antiperspirants, particularly inorganic and organic metal salts of the elements aluminium, zinc, magnesium, tin and zircon as well as their mixtures, wherein particularly halogenides such as aluminium chloride, alkaline aluminium hydroxychlorides, zirconyl oxychlorides and zirconyl hydroxychlorides as well as their mixtures are used. Often these aluminium and zirconium salts and mixtures thereof are also used in a complexed form, wherein as complex builders preferably propylene glycol, polyethylene glycol or glycine are used.

The present invention also relates to a method for producing a perfumed product, particularly a perfumed product according to the invention, comprising the following steps:
i) providing a compound according to the present invention or a perfume composition according to the present invention,
ii) providing one or several further components of the perfumed product to be produced, and
iii) contacting or mixing the further components provided in step ii) with a sensorially effective amount of the components provided in step i).

The present invention also relates to a method of imparting and/or increasing musk and/or floral odor characteristics to a perfumed product comprising the step of adding thereto a compound according to the invention or a perfume composition according to the invention.

In the following, the present invention will be illustrated in more detail by means of selected examples. Unless otherwise stated, all specifications thereby relate to the weight.

### Examples:

### Compound 1:

### 1-(3,3-dimethylcyclohexyl)ethyl 4-oxopentanoate

A mixture of Cyclodumol (10.0g, 64.1 mmol), Levulinic acid (8.9 g, 76.9 mmol) in toluene (50 mL) was added catalytic amount of pTs (0.52 g). This mixture was refluxed for about 5 h, after the reaction completion, at room temperature this reaction mixture was washed with water (2x 30ml) and the organic layer was concentrated on a rotary evaporator which gave crude material (17.5 g). Crude material was purified by Kügelrohr distillation (KRD)/bulb to bulb distillation, 160 °C, 1.8 mbar to provide 1-(3,3-Dimethylcyclohexyl)ethyl 4-oxopentanoate (14.6 g, 90%), as mixture of isomers. By PGC individual isomers were also identified and confirmed by NMR.

¹H NMR (600 MHz, Benzene-*d₆*) *δ* 4.86 (p, *J* = 6.3 Hz, 1H), 2.42-2.37 (m, 2H), 2.18 (td, *J* = 6.1, 1.2 Hz, 2H), 1.62 (s, 3H), 1.56 (dddd, *J* = 16.1, 6.7, 5.2, 3.4 Hz, 2H), 1.51-1.43 (m, 2H), 1.32 (tt, *J* = 13.3, 3.6 Hz, 1H), 1.29-1.23 (m, 1H), 1.11 (d, *J* = 6.4 Hz, 3H), 0.98 (td, *J* = 13.3, 4.2 Hz, 1H), 0.91 (s, 3H), 0.87 (d, *J* = 12.7 Hz, 1H), 0.84 (s, 3H), 0.77 (qd, *J* = 12.9, 3.9 Hz, 1H).

¹³C NMR (151 MHz, Benzene-*d₆*) *δ* 204.63, 172.08, 74.86, 41.48, 39.34, 38.66, 37.72, 33.71, 30.60, 29.17, 28.64, 28.44, 24.68, 22.30, 17.27.

### Compound 2:

### [2-[1-(3,3-dimethylcyclohexyl)ethoxy]-2-methyl-propyl] tetrahydrofuran-2-carboxylate

To a mixture of tetrahydrofuran-2-carboxylic acid (5.5 g, 47.4 mmol), 2-[1-(3,3-dimethylcyclohexyl)ethoxy]-2-methyl-propan-1-ol (*Lit:* EP1262474A1) (11.4 g, 2 50 mmol) in DCM (50 mL) was added 4-DMAP (0.9 g, 7.4 mmol) and by portion wise 1,3 Dicyclohexylcarbodiimide (DCC) (10.0 g, 49.0 mmol).This reaction mixture was stirred at room temperature overnight. This reaction mixture was filtered, washed with DCM (50 mL) and the filtrate was concentrated on a rotary evaporator to get crude product (16.9 g), which was purified by KRD, 115 °C, 0.7 mbar (14.1 g, 87%).

¹H NMR (400 MHz, Chloroform-d) *δ* 4.50 (dd, *J* = 8.4, 5.1 Hz, 1H), 4.07 - 3.98 (m, 3H), 3.98 - 3.89 (m, 1H), 3.42 - 3.33 (m, 1H), 2.26 (dtd, *J* = 12.0, 8.3, 6.3 Hz, 1H), 2.11 - 2.00 (m, 1H), 2.00 - 1.87 (m, 2H), 1.66 (dddd, *J =* 12.1, 5.2, 3.4, 1.7 Hz, 1H), 1.59 - 1.52 (m, 1H), 1.47 - 1.41 (m, 2H), 1.41 - 1.36 (m, 1H), 1.36 - 1.28 (m, 1H), 1.18 (s, 6H), 1.08 - 1.02 (m, 4H), 0.90 (s, 3H), 0.88 (s, 1H), 0.86 (d, *J* = 2.5 Hz, 3H), 0.83 - 0.75 (m, 1H).

¹³C NMR (101 MHz, CDCl₃) *δ* 173.29, 73.71, 71.78, 70.70, 69.34, 42.16, 40.38, 39.40, 33.71, 30.66, 30.26, 28.30, 25.24, 24.67, 23.97, 23.71, 22.30, 19.69.

### Compound 3:

### Dimethyl 2-butanoylbutanedioate (step 1):

A solution of Methyl-2-Chloracetat (6.2g, 56.7 mmol) und Methyl-3-Oxohexanoate (24.5 g, 170mmol) in THF (125 mL) was taken in a three-necked round bottom flask, equipped with a reflux condenser. To this reaction mixture, portionwise, was added potassium tert-butoxide (6.4 g, 56.7 mmol) (Exothermic: T<38°C) and after the addition, it was refluxed for 7 h. After the completion of the reaction, reaction mixture temperature was reduced to room temperature and quenched with cold water. Compound was extracted using MTBE (200 mL x 2) and the organic phase was washed with water (100 mL x 3), dried over sodium sulphate and the solvent was removed by evaporation (60 °C, 500-10 mbar) to yield the crude product Dimethyl 2-butanoylbutanedioate (11.5 g, 94%).

¹H NMR (400 MHz, Chloroform-*d*) *δ* 3.99 (dd, *J* = 8.3, 6.3 Hz, 1H), 3.75 (s, 3H), 3.68 (s, 3H), 3.04 - 2.79 (m, 2H), 2.75 - 2.57 (m, 2H), 1.64 (sextet, *J* = 7.4 Hz, 2H), 0.92 (t, *J* = 7.4 Hz, 3H).

¹³C NMR (101 MHz, CDCl₃) *δ* 203.82, 171.84, 168.97, 53.81, 52.72, 52.04, 44.64, 32.16, 16.87, 13.49.

### Methyl 4-oxoheptanoate (step 2):

A solution of Dimethyl 2-butanoylbutanedioate (8.4 g, 38.8 mmol) in water (20 mL) and methanol (2.2 mL) mixture, was taken in a three-necked round bottom flask, equipped with a reflux condenser. By dropwise, added NaOH solution 32% solution in water (12.1g, 97.7mmol) and refluxed for ~15 min and brought it to room temperature. To this reaction mixture was added H₂SO₄ (11.1g, 108.6mmol) and refluxed for ~15-30 min. After the completion of the reaction, at room temperature reaction mixture was further diluted with water (50 mL) and compound was extracted with MTBE (100 mL x2). Combined organic layer was washed with sat. NaHCO₃ solution, water (until neutral), dried over sodium sulphate and the solvent was removed by evaporation (60 °C, 500-10 mbar) to yield the crude product methyl 4-oxoheptanoate (6.4 g) Lit: Tetrahedron, 2008, 64 (51), 11713-11717).

¹H NMR (400 MHz, Chloroform-d) *δ* 3.68 (s, 3H), 2.74 - 2.69 (m, 2H), 2.61 - 2.56 (m, 2H), 2.43 (t, *J* = 7.4 Hz, 2H), 1.63 (sextet, *J* = 7.4 Hz, 2H), 0.92 (t, *J* = 7.4 Hz, 3H).

¹³C NMR (101 MHz, CDCl₃) *δ* 209.01, 173.33, 51.77, 44.69, 37.05, 27.71, 17.29, 13.71.

### 1-(3,3-dimethylcyclohexyl)ethyl 4-oxoheptanoate (step 3):

To a mixture of Zirkon-n-propylat (0.42 g) in Cyclodumol (4.7 g, 30.37 mmol), methyl 4-Oxoheptanoate (3.20 g, 20.25 mmol) was added. It was heated to 130-150°C, atm.press - 450 mbar for ~8h to get the crude product (6,3 g). Crude was purified by bulb-to bulb distillation (T = 180 °C, p = 0.80 mbar) gave 1-(3,3-dimethylcyclohexyl)ethyl 4-oxoheptanoate (2.88 g, 51%, GC purity 94,4%).

¹H NMR (400 MHz, Chloroform-*d*) *δ* 4.72 - 4.64 (m, 1H), 2.70 (t, *J* = 6.3 Hz, 2H), 2.56 (t, 2H), 2.43 (t, *J* = 7.3 Hz, 2H), 1.67 - 1.54 (m, 5H), 1.46 - 1.30 (m, 3H), 1.15 (d, *J* = 6.4 Hz, 3H), 1.07 (dd, *J* = 13.0, 4.1 Hz, 1H), 0.92 (t, 3H), 0.91 (s, 3H), 0.87 (s, 3H), 0.89 - 0.78 (m, 2H).

¹³C NMR (151 MHz, CDCl₃) *δ* 209.06, 172.48, 75.10, 44.74, 41.26, 39.11, 38.30, 37.16, 33.52, 30.52, 28.32, 28.30, 24.59, 21.96, 17.29, 17.06, 13.74.

### Compound 4:

### 1-(3,3-dimethylcyclohexyl)ethyl 4-oxooctanoate

### O1-ethyl O4-methyl 2-pentanoylbutanedioate (step 1):

A solution of Methyl-2-Chloracetat (4.6g, 42.4 mmol) und Methyl-3-Oxoheptanoate (18.4 g, 116.5 mmol) in THF (100 mL) was taken in a three-necked round bottom flask, equipped with a reflux condenser. To this reaction mixture, portion wise, was added potassium tert-butoxide (4.13 g, 36.8 mmol) (Exothermic: T<37°C) and after the addition, it was refluxed for 8-10 h. After the completion of the reaction, reaction mixture temperature was reduced to room temperature and quenched with cold water. Compound was extracted using MTBE (200 mL x 2) and the organic phase was washed with water (100 mL x 3), dried over sodium sulphate and the solvent was removed by evaporation (60 °C, 500-10 mbar) to yield the crude product, which was further purified by bulb-to bulb distillation (T = 90 °C, p = 0.88 mbar), afforded Dimethyl 2-pentanoylbutanedioate (7.4 g, 76%).

¹H NMR (600 MHz, Chloroform-d) *δ* 4.00 (dd, J = 8.3, 6.2 Hz, 1H), 3.75 (s, 3H), 2.98 (dd, J = 17.6, 8.3 Hz, 1H), 2.84 (dd, J = 17.6, 6.3 Hz, 1H), 2.71 (dt, J = 17.6, 7.5 Hz, 1H), 2.62 (dt, J = 17.6, 7.3 Hz, 1H), 1.59 (m_{c}, 2H), 1.37 - 1.28 (m, 2H), 0.91 (td, J = 7.4, 1.2 Hz, 6H).

¹³C NMR (151 MHz, CDCl₃) *δ* 203.97, 171.87, 169.00, 53.81, 52.74, 52.06, 42.50, 32.18, 25.47, 22.10, 13.82,

### methyl 4-oxooctanoate (step 2):

A solution of Dimethyl 2-pentanoylbutanedioate (7.3 g, 31.7 mmol) in water (15 mL) and methanol (1.5 mL) mixture, was taken in a three-necked round bottom flask, equipped with a reflux condenser. By dropwise, added 32% NaOH solution in water (7.3 g, 58.02 mmol) and refluxed for ~30 min and brought it to room temperature. To this reaction mixture was added H₂SO₄ (6.6 g, 64.98 mmol) and refluxed for ~30 min. After the completion of the reaction, at room temperature reaction mixture was further diluted with water (50 mL) and compound was extracted with MTBE (100 mL x2). Combined organic layer was washed with sat. NaHCOs solution, water (until neutral), dried over sodium sulphate and the solvent was removed by evaporation (60 °C, 500-10 mbar) to yield the crude product methyl 4-oxooctanoate (4.0 g, 73%, GC Purity 93%).

¹H NMR (600 MHz, Chloroform-d) *δ* 3.68 (s, 3H), 2.72 (td, J = 6.6, 4.7 Hz, 2H), 2.59 (t, J = 6.6 Hz, 2H), 2.45 (m, 2H), 1.58 (p, J = 7.5 Hz, 2H), 1.32 (m, 2H), 0.91 (t, J = 7.3 Hz, 3H).

¹³C NMR (151 MHz, CDCl₃) *δ* 209.32, 173.43, 51.81, 42.53, 37.02, 27.75, 25.92, 22.32, 13.83.

### 1-(3,3-dimethylcyclohexyl)ethyl 4-oxooctanoate (step 3):

To a mixture of Zirkon-n-propylat (0.48 g, 1.0 mmol) in Cyclodumol (4.8 g, 30.8 mmol), methyl 4-Oxooctanoate (4.0 g, 23.3 mmol) was added. It was heated to 140-160°C, atm.press - 380 mbar for ~8h to get the crude product, which was purified by bulb-to bulb distillation (T = 200 °C, p = 1.5-0.55 mbar) gave 1-(3,3-dimethylcyclohexyl)ethyl 4-oxooctanoate (5.2 g, 76%, GC purity 95%).

¹H NMR (400 MHz, Chloroform-*d*) *δ* 4.68 (t, 1H), 2.71 (t, 2H), 2.59-2.54 (m, 2H), 2.45 (t, 2H), 1.69-1.53 (m, 5H), 1.45-1.26 (m, 5H), 1.15 (d, *J =* 6.4 Hz, 3H), 1.10-1.02 (m, 1H), 0.94-0.88 (m, 6H), 0.88 (d, *J* = 2.5 Hz, 3H), 0.87-0.82 (m, 2H).

¹³C NMR (101 MHz, CDCl₃) *δ* 209.22, 172.50, 42.57, 41.26, 39.11, 38.30, 37.13, 33.52, 33.52, 30.52, 28.32, 28.32, 25.93, 24.59, 22.34, 21.96, 17.06, 13.85.

### Compound 5:

### 1-(3,3-dimethylcyclohexyl)ethyl 2-chloroacetate (step 1)

To a solution of Cyclodumol (63 g, 0.402 mol), Toluene (200 mL) and Pyridine (38 g, 0.482 mol) at 30 °C, a predissolved solution of Chloroacetyl chloride (50 g, 0.442 mol) in toluene (100 mL) was added dropwise (exothermic reaction). After the addition, the reaction temperature was maintained for another 2-3 h. The, reaction mixture was cooled 10-15 °C and quenched with ice cold water (100 mL) (slow addition). Organic phase was separated, organic layer washed with water (100 mL x 2), 5% soda solution (100 mL x 2) and evaporated (60°C/150-20mbar) to get the crude (99 g). Crude was purified using 5 cm Vigreux distillation (Flask temp = 94-120 °C, Head temp = 80-87 °C) to afford the title compound 1-(3,3-dimethylcyclohexyl)ethyl 2-chloroacetate (85 g, 91%). (*Lit:* EP1262474A1).

¹H NMR (400 MHz, Chloroform-d) *δ* 4.83 - 4.75 (m, 1H), 4.08 - 3.99 (m, 2H), 1.81 - 1.53 (m, 2H), 1.53 - 1.31 (m, 3H), 1.21 (dd, J = 6.4, 1.1 Hz, 3H), 1.11 - 0.99 (m, 2H), 0.91 (s, 3H), 0.88 (s, 3H), 0.87 -0.81 (m, 2H).

¹³C NMR (101 MHz, CDCl*₃*) *δ* 166.96, 77.29, 41.25, 41.18, 39.02, 38.27, 33.47, 30.52, 28.24, 24.57, 21.88, 16.96.

### [2-[1-(3,3-dimethylcyclohexyl)ethoxy]-2-oxo-ethyl] 4-oxopentanoate (step 2)

To a mixture of 1-(3,3-dimethylcyclohexyl)ethyl 2-chloroacetate (14.0 g, 60.3 mmol) and NMP (80 mL) was slowly added Luvilic acid (11,1 g, 95.7 mmol), slight exothermic was observed. Between 25-45°C, calcium carbonate (19,9 g, 14.4 mmol) was added portionwise. The reaction mixture was maintained at 55 °C. After the reaction is completed after 5 h, reaction mixture was quenched by slowly added water (200 ml) (observed exothermic). Compound was extracted with MTBE (50 ml x 3). Combined organic layer was washed with 5% NaCl solution (50 ml X2), dired over Na₂SO₄ and evaporated organic layer. To the crude, once again water (200 mL) water to remove the salt and extracted with Tolune (50 ml X3). Toluene was evaporated which gave 16 g of crude product, which upon purification by column chromatography (Cyclohexane: Ethylacetate, 95:5) gave the title compound (14.7 g, 78%).

¹H NMR (600 MHz, Chloroform-d) *δ* 4.78 (p, *J* = 6:3 Hz, 1H), 4.67 (td, *J* = 9.9, 1.4 Hz, 1H), 4.60 (s, 3H), 4.56 (d, *J* =15.8 Hz, 1H), 2.80 (td, *J* =7.0, 1.6 Hz, 4H), 2.73 - 2.67 (m, 4H), 2.20 (s, 3H), 2.19 (s, 3H), 1.86 - 1.75 (m, 2H), 1.72 (dd, *J* = 14.2, 10.0 Hz, 1H), 1.67 - 1.61 (m, 2H), 1.61 - 1.56 (m, 1H), 1.54 - 1.49 (m, 1H), 1.47 (ddd, *J* = 13.3, 10.8, 1.4 Hz, 2H), 1.44 - 1.39 (m, 1H), 1.39 - 1.32 (m, 3H), 1.25 - 1.20 (m, 1H), 1.18 (d, *J* = 6.4 Hz, 3H), 1.17 - 1.10 (m, 1H), 1.08 - 1.03 (m, 1H), 1.03 (s, 3H), 0.91 (s, 3H), 0.89 (s, 3H), 0.89 (d, *J* = 6.3 Hz, 3H), 0.87 (s, 3H), 0.86 - 0.80 (m, 2H).

¹³C NMR (151 MHz, CDCl₃) *δ* 206.31, 206.28, 172.16, 172.16, 167.45, 167.07, 77.97, 76.39, 61.05, 61.02, 47.16, 42.38, 41.13, 40.14, 39.04, 38.25, 37.86, 37.86, 36.18, 33.48, 31.46, 31.35, 30.51, 29.87, 29.86, 29.83 28.23, 27.63, 27.61, 24.56, 21.88, 21.61, 19.75, 17.00.

## Claims

1. Use of a compound selected from the group consisting of 1-(3,3-dimethylcyclohexyl)ethyl 4-oxopentanoate, 2-(1-(3,3-dimethylcyclohexyl)ethoxy)-2-methylpropyl tetra-hydrofuran-2-carboxylate, 1-(3,3-dimethylcyclohexyl)ethyl 4-oxoheptanoate, 1-(3,3-dimethylcyclohexyl)ethyl 4-oxooctanoate, 2-(1-(3,3-dimethylcyclohexyl)ethoxy)-2-oxoethyl 4-oxopentanoate,
as a perfuming ingredient.

2. Use according to claim 1, for enhancing, modifying or imparting a musk odor.

3. Use according to claim 2, for enhancing, modifying or imparting a fruity odor.

4. A compound selected from the group consisting of 1-(3,3-dimethylcyclohexyl)ethyl 4-oxopentanoate, 2-(1-(3,3-dimethylcyclohexyl)ethoxy)-2-methylpropyl tetra-hydrofuran-2-carboxylate, 1-(3,3-dimethylcyclohexyl)ethyl 4-oxoheptanoate, 1-(3,3-dimethylcyclohexyl)ethyl 4-oxooctanoate, 2-(1-(3,3-dimethylcyclohexyl)ethoxy)-2-oxoethyl 4-oxopentanoate.

5. Perfume composition comprising a compound as defined in claim 4.

6. Perfume composition according to claim 5 comprising one or more additional fragrance ingredients.

7. Perfumed product comprising a compound as defined in claim 4 or a perfume composition according to claim 5 or 6.

8. Perfumed product according to claim 7, wherein the product is selected from the group consisting of perfume extraits, eau de perfumes, eau de toilettes, aftershaves, eau de colognes, pre-shave products, splash colognes, perfumed refreshing tissues, acidic, alkaline and neutral detergents, textile fresheners, ironing aids, liquid detergents, powdery detergents, laundry pretreatment agents, fabric softeners, laundry soaps, laundry tablets, disinfectants, surface disinfectants, air improvers, aerosol sprays, waxes and polishes, personal care agents, hand creams and lotions, foot creams and lotions, depilatory creams and lotions, aftershave creams and lotions, tanning creams and lotions, hair care products, deodorants and antiperspirants, products for decorative cosmetic, candles, lamp oils, incense sticks, insecticides, repellents and fuels.

9. Method for producing a perfumed product, particularly a perfumed product according to claim 7 or 8, comprising the following steps:
i) providing a compound as defined in claim 4 or a perfume composition according to claim 5 or 6,
ii) providing one or several further components of the perfumed product to be produced, and
iii) contacting or mixing the further components provided in step ii) with a sensorially effective amount of the components provided in step i).

10. A method of imparting and/or increasing musk odor characteristics to a perfumed product comprising the step of adding thereto a compound as defined in claim 4 or a perfume composition as defined in claim 5 or 6.

## Patentansprüche

1. Verwendung einer Verbindung, ausgewählt aus der Gruppe bestehend aus 1-(3,3-Dimethylcyclohexyl)ethyl-4-oxopentanoat, 2-(1-(3,3-Dimethylcyclohexyl)ethoxy)-2-methylpropyl-tetrahydrofuran-2-carboxylat, 1-(3,3-Dimethylcyclohexyl)ethyl-4-oxoheptanoat, 1-(3,3-Dimethylcyclohexyl)ethyl-4-oxooctanoat, 2-(1-(3,3-Dimethylcyclohexyl)ethoxy)-2-oxoethyl-4-oxopentanoat,
als Duftstoffbestandteil.

2. Verwendung gemäß Anspruch 1 zur Verstärkung, Modifizierung oder Verleihung eines Moschusgeruchs.

3. Verwendung gemäß Anspruch 2 zur Verstärkung, Modifizierung oder Verleihung eines fruchtigen Geruchs.

4. Verbindung, ausgewählt aus der Gruppe bestehend aus 1-(3,3-Dimethylcyclohexyl)ethyl-4-oxopentanoat, 2-(1-(3,3-Dimethylcyclohexyl)ethoxy)-2-methylpropyl-tetrahydrofuran-2-carboxylat, 1-(3,3-Dimethylcyclohexyl)ethyl-4-oxoheptanoat, 1-(3,3-Dimethylcyclohexyl)ethyl-4-oxooctanoat, 2-(1-(3,3-Dimethylcyclohexyl)ethoxy)-2-oxoethyl-4-oxopentanoat.

5. Duftstoffzusammensetzung, umfassend eine Verbindung gemäß Anspruch 4.

6. Duftstoffzusammensetzung gemäß Anspruch 5, umfassend einen oder mehrere weitere Duftstoffbestandteile.

7. Parfümiertes Produkt, umfassend eine Verbindung gemäß Anspruch 4 oder eine Duftstoffzusammensetzung gemäß Anspruch 5 oder 6.

8. Parfümiertes Produkt gemäß Anspruch 7, wobei das Produkt ausgewählt ist aus der Gruppe bestehend aus Parfümextrakten, Eau de Parfums, Eau de Toilettes, Aftershaves, Eau de Colognes, Rasierwässern, Sprühparfüms, parfümierten Erfrischungstüchern, sauren, alkalischen und neutralen Reinigungsmitteln, Textilerfrischern, Bügelhilfen, Flüssigwaschmitteln, pulverförmigen Waschmitteln, Waschvorbehandlungsmitteln, Weichspülern, Waschseifen, Waschtabletten,
Desinfektionsmitteln, Flächendesinfektionsmitteln, Luft-erfrischern, Aerosolsprays, Wachsen und Polituren, Körperpflegemitteln, Handcremes und -lotionen, Fußcremes und -lotionen, Enthaarungscremes und -lotionen, Aftershave-Cremes und -lotionen, Bräunungscremes und -lotionen, Haarpflegeprodukten, Deodorants und Antitranspirantien, Produkte dekorativer Kosmetik, Kerzen, Lampenölen, Räucherstäbchen, Insektiziden, Repellentien und Brennstoffen.

9. Verfahren zur Herstellung eines parfümierten Produkts, insbesondere eines parfümierten Produkts gemäß Anspruch 7 oder 8, umfassend die folgenden Schritte:
i) Bereitstellung einer Verbindung gemäß Anspruch 4 oder einer Duftstoffzusammensetzung gemäß Anspruch 5 oder 6,
ii) Bereitstellung eines oder mehrerer weiterer Bestandteile des herzustellenden parfümierten Produkts, und
iii) Zusammenführen oder Vermengen der in Schritt ii) bereitgestellten weiteren Bestandteile mit einer sensorisch wirksamen Menge der in Schritt i) bereitgestellten Komponenten.

10. Verfahren zur Verleihung und/oder Verstärkung moschusartiger Duftcharakteristika eines parfümierten Produkts, umfassend den Schritt des Hinzufügens einer Verbindung gemäß Anspruch 4 oder einer Duftstoffzusammensetzung gemäß Anspruch 5 oder 6.

## Revendications

1. Utilisation d'un composé choisi dans le groupe constitué par le 4-oxopentanoate de 1-(3,3-diméthylcyclohexyl)éthyle, le tétrahydrofurane-2-carboxylate de 2-(1-(3,3-diméthylcyclohexyl)éthoxy)-2-méthylpropyle, le 4-oxoheptanoate de 1-(3,3-diméthylcyclohexyl)éthyle, le 4-oxooctanoate de 1-(3,3-diméthylcyclohexyl)éthyle, le 4-oxopentanoate de 2-(1-(3,3-diméthylcyclohexyl)éthoxy)-2-oxoéthyle, comme ingrédient parfumant.

2. Utilisation selon la revendication 1, pour intensifier, modifier ou conférer une odeur de musc.

3. Utilisation selon la revendication 2, pour intensifier, modifier ou conférer une odeur fruitée.

4. Composé choisi dans le groupe constitué par le 4-oxopentanoate de 1-(3,3-diméthylcyclohexyl)éthyle, le tétrahydrofurane-2-carboxylate de 2-(1-(3,3-diméthylcyclohexyl)éthoxy)-2-méthylpropyle, le 4-oxoheptanoate de 1-(3,3-diméthylcyclohexyl)éthyle, le 4-oxooctanoate de 1-(3,3-diméthylcyclohexyl)éthyle, le 4-oxopentanoate de 2-(1-(3,3-diméthylcyclohexyl)éthoxy)-2-oxoéthyle.

5. Composition de parfum comprenant un composé tel que défini dans la revendication 4.

6. Composition de parfum selon la revendication 5, comprenant un ou plusieurs ingrédients de parfum supplémentaires.

7. Produit parfumé comprenant un composé tel que défini dans la revendication 4 ou une composition de parfum selon la revendication 5 ou 6.

8. Produit parfumé selon la revendication 7, dans lequel le produit est choisi dans le groupe constitué par les extraits de parfum, les eaux de parfum, les eaux de toilette, les après-rasages, les eaux de Cologne, les produits de pré-rasage, les eaux de Cologne splash, les lingettes rafraîchissantes parfumées, les détergents acides, alcalins et neutres, les assainisseurs de textiles, les aides au repassage, les détergents liquides, les détergents en poudre, les agents de prétraitement du linge, les assouplissants, les savons de lavage, les pastilles de lavage, les désinfectants, les désinfectants de surface, les assainisseurs d'air, les sprays aérosols, les cires et les produits de cirage, les produits de soins personnels, les crèmes et lotions pour les mains, les crèmes et lotions pour les pieds, les crèmes et lotions dépilatoires, les crèmes et lotions après-rasage, les crèmes et lotions de bronzage, les produits de soins capillaires, les déodorants et les antiperspirants, les produits cosmétiques décoratifs, les bougies, les huiles de lampe, les bâtons d'encens, les insecticides, les répulsifs et les carburants.

9. Procédé de production d'un produit parfumé, en particulier d'un produit parfumé selon la revendication 7 ou 8, comprenant les étapes suivantes consistant à:
i) fournir un composé tel que défini dans la revendication 4 ou une composition de parfum selon la revendication 5 ou 6,
ii) fournir un ou plusieurs autres composants du produit parfumé à produire, et
iii) mettre en contact ou mélanger les autres composants fournis à l'étape ii) avec une quantité sensoriellement efficace des composants fournis à l'étape i).

10. Procédé destiné à conférer et/ou à intensifier des caractéristiques olfactives musquées à/d'un produit parfumé, comprenant l'étape consistant à ajouter un composé tel que défini dans la revendication 4 ou une composition de parfum telle que définie dans la revendication 5 ou 6.
